# EUROPEAN PATENT SPECIFICATION

(11) **EP 1 155 681 B1**
(45) Date of publication and mention of the grant of the patent: **21.05.2008**
(21) Application number: 01203156.3
(22) Date of filing: 18.08.1995
(51) Int. Cl.: A61K 8/38, A61K 8/44, A61Q 11/00

(54) **Oral composition with teeth whitening effect**
Orale Formulierung zum Bleichen von Zähnen
Composition orale pour le blanchiment des dents

(30) Priority: 22.08.1994 EP 94306191
(43) Date of publication of application: 21.11.2001
(62) Divisional of application: 95930506.1
(73) Proprietor: UNILEVER N.V., 3013 AL Rotterdam (NL); UNILEVER PLC, London EC4P 4BQ (GB)
(72) Inventor: Thornthwaite, David William, Unilever Research, Bebington, Wirral, Merseyside CH63 3JW (GB); Joiner, Andrew, Bebington, Wirral, CH63 3JW (GB)
(74) Representative: Whaley, Christopher

(56) References cited:
- EP-A- 0 228 137
- EP-A- 0 349 220
- EP-A- 0 545 594
- EP-A- 0 564 251
- US-A- 3 988 433
- US-A- 5 098 598
- US-A- 5 220 052

## Description

The present invention relates to an oral composition with an improved teeth whitening effect. More particularly, it relates to an oral composition with an improved teeth whitening effect comprising a safe and effective amount of a certain organic peroxyacid.

The use of peroxy compounds in oral care compostions has already been proposed in the prior art. Many peroxy compounds have been suggested for whitening/bleaching human teeth, and representative examples of such peroxy compounds are hydrogen peroxide, urea peroxide, organic peracids such as perphthalic acid, diperoxycarboxylic acids, 1,12-dodecanedioic peroxy acid, peroxy acetic acid and systems comprising a peroxy compound and a peroxy acid precursor which generate peroxy acetic acid in situ, such as sodium perborate and tetraacetylethylene diamine (TAED). The use of peroxy acetic acid is suggested in particular in e.g. EP-A-0545,594 (Colgate), which also sets out the various prior proposals, made in the art for several peroxy compounds as bleaching/whitening agent for human teeth.

We have now found that a certain organic peroxy acid, which will be defined hereinafter, is either more stable than peroxy acetic acid or is more effective than peroxy acetic acid. This organic peroxy acid is monononylamide of peroxyadipic acid ("NAPAA") which is described in EP-A-349,220.

Thus, the present invention relates to an oral composition with teeth whitening effect, comprising a safe and effective amount of an organic peroxy acid as bleaching/whitening agent, characterised in that the organic peroxy acid is a monononylamide of peroxyadipic acid.

The amount of peroxyacid, used in the present invention, may vary from 0.01 to 99 % by weight of the oral composition, preferably from 0.1 to 30 % by weight, particularly preferably from 0.1-5 % by weight.

The oral compositions can be formulated in any suitable application form, such as gels, mouthwashes, toothpowders and toothpastes. They may be formulated into a single formulation or they may be formulated for multi compartment containers into different formulations, e.g. one containing the peroxy bleach and ingredients compatible therewith, and another containing the remaining ingredients.

The oral care compositions of the present invention may furthermore comprise optional, conventional ingredients such as pharmaceutically acceptable carriers like starch, sucrose, water or water/alcohol systems etc.. Small amounts of surfactants may also be included, such as anionic, nonionic and amphoteric surfactants. When formulated into a dentifrice, such formulation may contain all the usual dentifrice ingredients.

Thus, they may comprise particulate abrasive materials such as silicas, aluminas, calcium carbonates, dicalciumphosphates, calcium pyrophosphates hydroxyapatites, trimetaphosphates, insoluble hexametaphosphates and so on, usually in amounts between 5 and 60 % by weight.

Furthermore, the dentifrice formulations may comprise humectants such as glycerol, sorbitol, propyleneglycol, xylitol, lactitol and so on.

Binders and thickeners such as sodium carboxymethylcellulose, xanthan gum, gum arabic etc. may also be included, as well as synthetic polymers such as polyacrylates and carboxyvinyl polymers such as Carbopol^{®}.

Flavours such as peppermint and spearmint oils may also be included, as well as preservatives, opacifying agents, colouring agents, pH-adjusting agents, sweetening agents and so on. Stabilising agents for the organic peroxy acids such as dipicolinic acid or sodium stannate may also be usefully included.

Anti-bacterial agents may also be included such as Triclosan, chlorhexidine, copper-, zinc- and stannous salts such as zinc citrate, sodium zinc citrate and stannous pyrophosphate, sanguinarine extract, metronidazole. Further examples of anti-bacterial agents are quaternary ammonium compounds such as cetylpyridinium chloride; bis-guanides such as chlorhexidine digluconate, hexetidine, octenidine, alexidine; halogenated bisphenolic compounds such as 2,2' methylenebis-(4-chloro-6-bromophenol).

Polymeric compounds which can enhance the delivery of active ingredients such as anti-bacterial agents can also be included. Examples of such polymers are copolymers of polyvinylmethylether with maleic anhydride and other similar delivery enhancing polymers, e.g. those described in DE-A-3,942,643 (Colgate).

Furthermore anti-inflammatory agents such as ibuprofen, flurbiprofen, aspirin, indomethacin etc. may also be included.

Anti-caries agents such as sodium- and stannous fluoride, aminefluorides, monosodiumfluorophosphate, casein, plaque buffers such as urea, calcium lactate, calcium glycerophosphate, strontium polyacrylates may also be included. Other optional ingredients include vitamins such as Vitamin C, and plant extracts. Desensitising agents such as potassium citrate, potassium chloride, potasium tartrate, potassium bicarbonate, potassium oxalate, potassium nitrate as well as strontium salts may also be included.

Buffers and salts to buffer the pH and ionic strength of the compositions may also be included. Liposomes and other encapsulates may also be used to improve delivery or stability of active ingredients.

Furthermore, the oral compositions may comprise anti-calculus agents such as alkalimetal pyrophosphates, hypophosphite-containing polymers, organic phosphonates, phosphocitrates etc..

In addition, the compositions may comprise functional biomolecules such as bacteriocins, antibodies, enzymes and so on.

Other optional ingredients that may be included are e.g. bleaching agents, e.g. those described in EP-A-0 545,594, effervescing systems such as sodium bicarbonate/citric acid systems, colour change systems, and so on.

When formulated as a mouthwash, the oral care composition usually comprises a water/alcohol solution, flavour, humectant, sweetener and colorant.

Since the peroxyacid of the invention also has an antimicrobial property, the composition of the invention is also effective to combat plaque and caries.

The present invention will further be illustrated by way of Example.

### Example I

The bleaching agents were evaluated as follows:
(1) Synthetic hydroxyapatite discs were polished and placed in sterile saliva at 37°C overnight to form a pellicle.
(2) Discs were stained with tea/coffee/iron salts/saliva mixture for seven days at 37°C.
(3) Stained discs were immersed in bleaching solutions for desired time.
(4) The change in colour of the discs was measured using a Minolta chromameter CR-300 in L*a*b* mode. Using L* (initial), L* (soiled), and L* (cleaned), the percentage of stain removed was calculated.

### Example II

1. Various peracids were tested according to the method of Example I.
The concentrations of peracids were all 36mM made up in 0.5M sodium bicarbonate.

| Time (mins) | % stain removed with | | | |
|---|---|---|---|---|
| | BIPTA | DPDDA | NAPAA | PAP |
| 0 | 0 | 0 | 0 | 0 |
| 1 | 25 | 16 | 16 | 13 |
| 3 | 39 | 32 | 29 | 28 |
| 5 | 51 | -- | 40 | 39 |

| | | | | |
|---|---|---|---|---|
| BIPTA = butyl imido peroxytrimellitic acid not according to the invention DPDDA = 1,10 Diperoxydodecandioic acid (for comparison; DPDDA is not according to the invention) NAPAA = monononylamide of peroxyadipic acid PAP = N-phthalimido hexanoic acid not according to the invention. | | | | |

## Claims

1. An oral composition with teeth whitening effect, comprising a safe and effective amount of an organic peroxy acid, **characterised in that** the organic peroxy acid is a monononylamide of peroxyadipic acid ("NAPAA").

2. Use of the peroxyacids of claim 1 as teeth whitening agent in the manufacture of an oral composition having teeth whitening properties.

## Patentansprüche

1. Orale Zusammensetzung mit Bleichwirkung für Zähne, umfassend eine sichere und wirksame Menge einer organischen Peroxysäure, **dadurch gekennzeichnet, dass** die organische Peroxysäure ein Monononylamid von Peroxyadipinsäure ("NAPAA") ist.

2. Verwendung der Peroxysäuren von Anspruch 1 als Bleichmittel für Zähne bei der Herstellung einer oralen Zusammensetzung, die Bleichwirkung für Zähne hat.

## Revendications

1. Composition orale avec un effet de blanchiment des dents, comprenant une quantité sûre et efficace d'un peroxyacide organique, **caractérisée en ce que** le peroxyacide organique est un monononylamide d'acide peroxyadipique ("NAPAA").

2. Utilisation des peroxyacides selon la revendication 1 en tant qu'agent de blanchiment des dents dans la fabrication d'une composition orale ayant des propriétés de blanchiment des dents.
